(19) Europäisches Patentamt / European Patent Office / Office européen des brevets

(11) **EP 3 343 240 B1**

(12) **FASCICULE DE BREVET EUROPEEN**

(45) Date de publication et mention
de la délivrance du brevet:
**21.08.2019 Bulletin 2019/34**

(51) Int Cl.:
***G01R 33/00*** *(2006.01)*   ***A61B 5/06*** *(2006.01)*
***G01V 3/08*** *(2006.01)*

(21) Numéro de dépôt: **17210724.5**

(22) Date de dépôt: **27.12.2017**

(54) **RÉSEAU DE MAGNÉTOMÈTRES VECTORIELS ET PROCÉDÉ DE LOCALISATION ASSOCIÉ**

VEKTORMAGNETOMETER-NETZ UND ENTSPRECHENDES LOKALISIERUNGS-VERFAHREN

NETWORK OF VECTOR MAGNETOMETERS AND CORRESPONDING LOCALISATION METHOD

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.01.2017 FR 1750010**

(43) Date de publication de la demande:
**04.07.2018 Bulletin 2018/27**

(73) Titulaire: **Commissariat à l'énergie atomique
et aux énergies alternatives
75015 Paris (FR)**

(72) Inventeurs:
• **LE PRADO, Matthieu
 38160 SAINT-MARCELLIN (FR)**
• **ALOUI, Saifeddine
 38600 FONTAINE (FR)**
• **LABYT, Etienne
 38950 SAINT-MARTIN-LE-VINOUX (FR)**

(74) Mandataire: **Brevalex
 95, rue d'Amsterdam
 75378 Paris Cedex 8 (FR)**

(56) Documents cités:
**EP-B1- 1 272 862    US-A1- 2013 249 784**

**Description**

**DOMAINE TECHNIQUE**

Le domaine de l'invention est celui de l'imagerie des champs biomagnétiques, et concerne plus particulièrement les réseaux de magnétomètres vectoriels utilisés notamment en magnétocardiographie ou en magnétoencéphalographie.

**ÉTAT DE LA TECHNIQUE ANTÉRIEURE**

Des magnétoencéphalographes aujourd'hui utilisés exploitent des magnétomètres de technologie SQUID (« Superconducting Quantum Interference Device ») qui sont inclus dans un support rigide, de type Dewar, qui contient également des liquides cryogéniques. L'inconvénient de ces dispositifs est que leur géométrie immuable est conçue pour pouvoir accueillir les plus grosses têtes. Les magnétomètres répartis notamment sur les côtés d'un casque du magnétoencéphalographe restent donc distants du cerveau des personnes ayant de petites têtes, en particulier les enfants. Il en découle des performances limitées.

Les réseaux de magnétomètres vectoriels, en particulier les magnétomètres à pompage optique, ne nécessitent pas de cryogénie, et permettent de concevoir des magnétoencéphalographes portés par une structure conformable, i.e. de géométrie variable, à même de pouvoir s'adapter aux différentes morphologies (ici différentes formes de tête) de patient.

Mais afin de pouvoir interpréter correctement les données récoltées par un tel réseau de magnétomètres, il faut être en mesure d'avoir une connaissance préalable de la localisation, en position et en orientation, des magnétomètres selon la géométrie que le support conformable va adopter pour s'adapter au patient.

**EXPOSÉ DE L'INVENTION**

L'invention vise à répondre à ce besoin, et propose pour ce faire un procédé défini par la revendication 1 pour la localisation d'au moins un magnétomètre vectoriel, notamment un magnétomètre d'un appareil de magnétocardiographie ou de magnétoencéphalographie. Le procédé comprend les étapes suivantes :

- génération, par une source de champ magnétique, de m champs magnétiques de référence, m étant un entier supérieur ou égal à 2, les amplitudes des m champs magnétiques étant connues et les directions des m champs magnétiques étant connues et distinctes ;
- mesure des m champs magnétiques de référence suivant n axes de l'au moins un magnétomètre vectoriel, n étant un entier supérieur ou égal à 2 et m et n étant tels que m*n$\geq$6 ;
- détermination de la position et de l'orientation, par rapport à la source de champ magnétique, de l'au moins un magnétomètre vectoriel à partir de la mesure des m champs magnétiques de référence sur les n axes de l'au moins un magnétomètre vectoriel.

Certains aspects préférés mais non limitatifs de ce procédé sont les suivants :

- les m champs magnétiques de référence sont émis simultanément, les amplitudes des m champs magnétiques étant distinctes ;
- les m champs magnétiques de référence sont émis séquentiellement ;
- l'étape de génération comprend un créneau temporel de non-génération d'un champ magnétique de référence, une mesure du champ magnétique ambiant suivant les n axes de l'au moins un magnétomètre vectoriel à localiser est réalisée au cours dudit créneau temporel, et l'étape de détermination comprend une soustraction de la mesure du champ magnétique ambiant à la mesure des m champs magnétiques de référence ;
- la source de champ magnétique réalise une modulation fréquentielle des m champs magnétiques de référence ;
- la source de champ magnétique réalise un multiplexage fréquentiel des m champs magnétiques de référence ;
- la source de champ magnétique comprend m bobines et un générateur de fréquence permettant d'injecter un courant d'amplitude connue dans chacune des m bobines ;
- l'au moins un magnétomètre vectoriel à localiser appartient à un réseau de magnétomètres vectoriels, et la source de champ magnétique est une source externe audit réseau ;
- l'au moins un magnétomètre vectoriel à localiser appartient à un réseau de magnétomètres vectoriels, et la source de champ magnétique est l'un des magnétomètres vectoriels dudit réseau ;
- les étapes de génération, mesure et détermination sont réitérées en utilisant pour source de champ magnétique un autre magnétomètre vectoriel dudit réseau ;

- les étapes de mesure et de détermination sont mises en oeuvre par le ou les magnétomètres du réseau situés dans une zone d'émission autour de la source de champ magnétique, et ledit autre magnétomètre est choisi parmi ledit ou lesdits magnétomètres du réseau situés dans la zone d'émission autour de la source de champ magnétique ;
- l'au moins un magnétomètre vectoriel est porté par une structure conformable et le procédé comprend une étape préalable d'installation de la structure conformable sur un utilisateur.

L'invention s'étend à un appareil de mesure configuré pour mettre en oeuvre ce procédé. Cet appareil est défini par la revendication 13.

## BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

- la figure 1 est un schéma d'un mode de réalisation possible du procédé conforme à l'invention ;
- la figure 2 est un schéma illustrant la localisation de magnétomètres récepteurs relativement à un magnétomètre émetteur;
- la figure 3 illustre une localisation de proche en proche, avec recouvrement, pouvant être réalisée dans le cadre de l'invention.

## EXPOSÉ DÉTAILLÉ DE MODES DE RÉALISATION PARTICULIERS

L'invention porte sur un appareil de mesure de champ magnétique, notamment un appareil trouvant application dans le domaine médical pour l'imagerie des champs biomagnétiques, tel qu'un appareil de magnétoencéphalographie ou de magnétocardiographie et plus particulièrement un appareil doté de magnétomètres vectoriels agencés en réseau. L'invention porte plus particulièrement sur un appareil configuré pour permettre la mise en oeuvre du procédé de localisation des magnétomètres décrit ci-après.

Le réseau de magnétomètres vectoriels est typiquement porté par une structure conformable destinée à être portée par un utilisateur en s'adaptant à sa morphologie. En magnétoencéphalographie, la structure conformable prend la forme d'un casque à même d'épouser la forme du crâne de l'utilisateur sur lequel il est installé, chaque magnétomètre se retrouvant directement en contact avec le crâne.

Le procédé selon l'invention comprend ainsi une étape préalable d'installation de la structure conformable sur l'utilisateur. La localisation des différents magnétomètres dépend ainsi de la morphologie de l'utilisateur, et il convient donc de disposer d'une connaissance de cette localisation avant d'utiliser l'appareil pour l'imagerie des champs biomagnétiques.

Les magnétomètres vectoriels du réseau permettent une mesure du champ magnétique suivant une direction physique. Il s'agit par exemple de magnétomètres à pompage optique. Ces magnétomètres emploient une cellule remplie d'un gaz d'atomes, une source laser qui émet un faisceau polarisé en direction de la cellule, ainsi qu'un photo-détecteur apte à délivrer un signal de sortie représentatif du faisceau ayant traversé la cellule. Au moins une bobine, trois typiquement, entoure la cellule, et est alimentée par un générateur de fréquence pour générer un champ magnétique d'excitation sinusoïdal, perpendiculaire à la direction de polarisation des photons issus du laser s'ils sont polarisés rectilignement et perpendiculaire au faisceau laser s'ils sont polarisés circulairement. Le photo-détecteur permet de mesurer l'amplitude du signal de sortie à un harmonique de la fréquence d'oscillation du champ magnétique d'excitation. Cette amplitude est directement proportionnelle au champ à mesurer, tout du moins lorsque celui-ci est suffisamment faible, ce qui est le cas pour les applications visées pour lesquelles l'amplitude des signaux biomagnétiques issus du coeur ou du cerveau est inférieure à 1 nT.

Ces bobines peuvent être utilisées pour générer des champs magnétiques d'amplitudes et directions connues, c'est-à-dire de moment magnétique $\vec{m}$ connu. Dans un cas parfait, une bobine génère un champ magnétique dipolaire $\vec{B}$ et ce champ magnétique dipolaire de moment magnétique $\vec{m}$ mesuré à une position relative $\vec{r}$ admet la valeur suivante:

$$\vec{B} = \frac{\mu_0}{4\pi d^5}\left(3(\vec{m}\cdot\vec{r})\vec{r} - d^2\vec{m}\right),$$ avec $\mu_0$ la perméabilité magnétique du vide et d la distance entre la bobine et la position relative. On notera qu'un modèle plus avancé de cette mesure du champ $\vec{B}$ à la position relative $\vec{r}$ peut être développé à partir des équations de Biot et Savart.

L'invention exploite cette possibilité afin de déterminer la position et l'orientation relative des différents magnétomètres du réseau. Elle utilise l'un des magnétomètres du réseau en tant qu'émetteur de champ magnétique de référence (en variante une source externe au réseau peut être utilisée) pendant que les autres magnétomètres mesurent le champ magnétique émis. On cherche alors à remonter à la position et l'orientation des magnétomètres de mesure par rapport au magnétomètre émetteur du champ magnétique de référence.

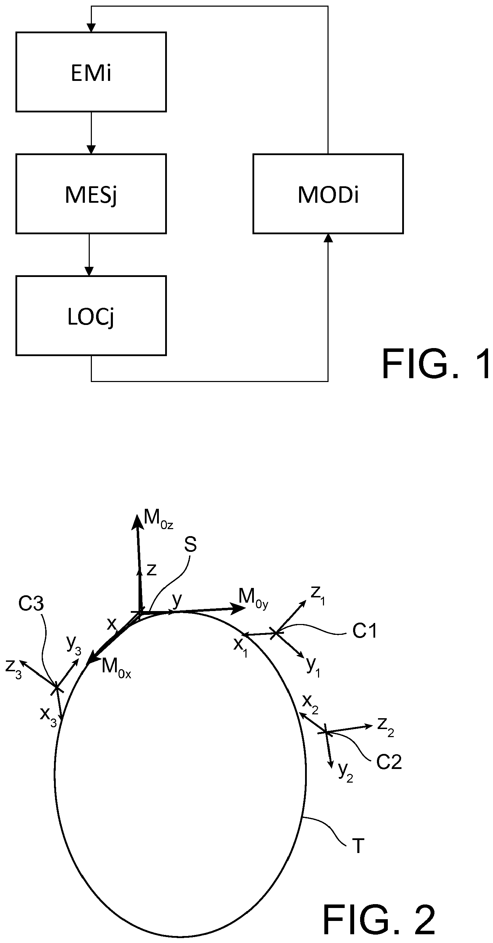

En référence à la figure 1, l'invention porte ainsi sur un procédé de localisation d'au moins un magnétomètre vectoriel, qui comprend les étapes suivantes.

Une première étape comprend la génération, par une source de champ magnétique, de m champs magnétiques de référence, m étant un entier supérieur ou égal à 2, les amplitudes des m champs magnétiques étant connues et les directions des m champs magnétiques étant connues et distinctes.

Une deuxième étape comprend la mesure des m champs magnétiques de référence suivant n axes de l'au moins un magnétomètre vectoriel à localiser, n étant un entier supérieur ou égal à 2 et m et n étant tels que m*n≥6.

Une troisième étape comprend la détermination de la position et de l'orientation, par rapport à la source de champ magnétique, de l'au moins un magnétomètre vectoriel à partir de la mesure des m champs magnétiques de référence sur les n axes de l'au moins un magnétomètre vectoriel.

On retiendra que pour déterminer la position et l'orientation des magnétomètres par rapport à la source de champ magnétique, au moins 6 mesures décorrélées sont nécessaires. Pour ce faire la source génère m ≥ 2 champs magnétiques dont les moments magnétiques équivalents sont dirigés dans des directions distinctes, ces champs étant mesurées sur plusieurs, n ≥ 2, axes de chaque magnétomètre de sorte que m x n fasse au moins 6. Un nombre supérieur de mesures permet d'avoir de la redondance et apporter plus d'information ainsi que filtrer le bruit de mesure.

L'invention exploite ainsi une source de champ magnétique qui peut générer des champs magnétiques décrits dans un référentiel local lié à la source, et plus particulièrement une source de champ magnétique qui comprend m bobines et un générateur de fréquence permettant d'injecter un courant d'amplitude connue dans chacune des m bobines. Typiquement, on a recours à une source tri-axes qui émet trois champs magnétiques, soit de façon simultanée soit de façon séquentielle.

Le ou les magnétomètres à localiser sont des entités capables de mesurer une ou plusieurs composantes d'un champ magnétique. Typiquement, un magnétomètre tri-axes mesure la projection du vecteur champ magnétique sur trois axes décrits dans un référentiel local au magnétomètre.

On pose $\vec{x}_j^i$ le vecteur d'état à estimer décrivant la position et orientation du magnétomètre à localiser d'indice j dans le référentiel de la source d'indice i. Ce vecteur peut être composé par exemple de 6 ou 7 paramètres :

- 3 coordonnées cartésiennes (ou autre forme de coordonnées : cylindriques, sphériques...) définissant la position du magnétomètre à localiser par rapport à la source ;
- 3 angles de rotation ou autre forme équivalente décrivant une rotation (par exemple 3 coordonnées de l'axe de révolution + angle de rotation, ou bien un quaternion ...).

On pose $\vec{B}_j^i = \vec{h}(\vec{x}_j^i)$ l'équation décrivant la mesure du/des champs magnétique(s) de référence émis par la source i et mesuré(s) par le magnétomètre j. Lors de l'étape de localisation LOCj, on vient inverser la fonction $\vec{h}(\vec{x}_j^i)$ afin d'estimer le vecteur d'état $\vec{x}_j^i$ à partir des mesures $\vec{B}_j^i$.

Dans un premier mode de réalisation possible, l'au moins un magnétomètre vectoriel à localiser appartient à un réseau de magnétomètres vectoriels, et la source de champ magnétique est une source externe audit réseau. Cette source peut notamment comprendre au moins m bobines englobant le réseau et un générateur de fréquence permettant d'injecter un courant d'amplitude connue dans les m bobines.

Dans un second mode de réalisation de l'invention, l'au moins un magnétomètre vectoriel à localiser appartient à un réseau de magnétomètres vectoriels, et la source de champ magnétique est l'un des magnétomètres du réseau. Dans ce second mode de réalisation, on utilise m bobines déjà présentes sur les magnétomètres pour opérer l'un de ceux-ci en tant que source de champ magnétique et un générateur de fréquence permettant d'injecter un courant d'amplitude connue dans chacune de ces m bobines.

Les magnétomètres à pompage optique emploient effectivement des bobines pour générer des champs magnétiques basse fréquence, par exemple dans la gamme [0-300] Hz. Ils sont par exemple équipés de bobines de Helmholtz de 1 cm de diamètre, dont chaque spire est composée d'un seul fil parcouru par un courant i de 1 mA. Leur moment magnétique est $\vec{M}_0 = 2.S.i\ \vec{U}_z = 1{,}57\ 10\text{-}7\ A.m^2$ selon l'axe $\vec{U}_z$. Ce courant permet de générer des champs magnétiques d'amplitude supérieure ou égale à 2 nT et 2 pT à 2 et 20 cm respectivement, qui sont facilement détectables par des magnétomètres à pompage optique qui sont affectés par un bruit de 200 fT/√Hz de 1 à 100 Hz. Dans les applications visées, les magnétomètres à localiser sont placés à moins de 20 cm du magnétomètre source. Tous les magnétomètres du réseau peuvent ainsi détecter le champ magnétique généré par la source et être localisés.

Dans ce cadre, on notera que la disposition des magnétomètres du réseau permet une localisation dont les effets des perturbations, d'origine basse fréquence pour la plupart, peuvent être minimisés en utilisant une bande dépassant 10 Hz pour éviter le champ ambiant et au-dessous de 100 Hz pour limiter les champs de Foucault. Ces magnétomètres peuvent en outre émettre des champs magnétiques très faibles en intensité favorisant la possibilité d'effectuer une localisation séquentielle des magnétomètres telle que décrite par la suite.

La figure 2 illustre la localisation de magnétomètres récepteurs C1-C3 relativement à un magnétomètre émetteur S, ces différents magnétomètres C1-C3, S appartenant à un réseau de magnétomètres porté par un casque conformable installé sur la tête T d'un utilisateur. Le magnétomètre émetteur S est contrôlé pour émettre un champ magnétique de référence de moment magnétique connu, d'amplitude $M_{ox}$, $M_{oy}$, $M_{oz}$ sur chacun des axes x, y et z du référentiel associé au magnétomètre émetteur S. Le procédé selon l'invention permet de déterminer la position et l'orientation, par rapport au magnétomètre émetteur S, des magnétomètres à localiser C1-C3.

Lors de l'étape de génération EMi des m champs magnétiques de référence de moments magnétiques connus et dirigés selon des directions distinctes, ces m champs peuvent être générés de façon séquentielle. Ces champs sont ainsi générés tour à tour et individuellement. Chaque champ étant généré de façon continue dans un intervalle temporel fixe qui lui est dédié, on peut parler de multiplexage temporel.

L'environnement de l'appareil de mesure peut être blindé, de manière à éliminer le champ ambiant. En variante, l'environnement de l'appareil de mesure peut ne pas être blindé et le multiplexage temporel comprend alors un créneau temporel de non-génération d'un champ magnétique de référence. La mesure du champ magnétique ambiant suivant les n axes d'un magnétomètre vectoriel à localiser est alors réalisée au cours dudit créneau temporel, et la détermination de la position et de l'orientation, par rapport à la source de champ magnétique, du magnétomètre vectoriel comprend une soustraction de la mesure du champ magnétique ambiant à la mesure des champs magnétiques de référence.

Les champs générés ne sont pas nécessairement continus, l'invention s'étendant à la génération de champs magnétiques de référence variables, par exemple au moyen d'une modulation fréquentielle des champs magnétiques de référence introduite par la source de champ magnétique. L'amplitude et la phase du signal reçu par un magnétomètre à localiser permettent de déterminer le vecteur champ magnétique équivalent. Cette technique permet ainsi d'éliminer le champ ambiant sans avoir besoin de blindage. Dans ce cadre, les champs magnétiques de référence peuvent être générés de façon séquentielle comme présenté précédemment. Alternativement, la source de champ magnétique réalise un multiplexage fréquentiel des m champs magnétiques de référence, ceux-ci étant générés simultanément en exploitant des fréquences porteuses différentes sur chacun des m axes.

Une génération simultanée des m champs magnétique de référence peut également être réalisée en réalisant une modulation d'amplitude desdits champs pour que les amplitudes des m champs magnétiques soient distinctes.

La direction d'un champ magnétique de référence n'est en outre pas nécessairement continue. Il est ainsi possible de faire varier cette direction, par exemple jusqu'à trouver la direction qui maximise l'amplitude du champ mesuré par un magnétomètre à localiser.

Dans le cadre du second mode de réalisation dans lequel l'un des magnétomètres du réseau est utilisé en tant que source de champ magnétique, il est possible de venir réitérer les étapes de génération, mesure et détermination en utilisant pour source de champ magnétique un autre magnétomètre vectoriel dudit réseau. Ainsi plusieurs magnétomètres du réseau peuvent à tour de rôle être utilisés pour générer les champs de référence. Cette réitération est illustrée sur la figure 1 par le bloc « MODi » qui représente le changement de magnétomètre destiné à être utilisé comme source de champs de référence lors de l'étape EMi.

Dans le cadre de cette variante, et après un nombre de réitération donné, par exemple égal au nombre de magnétomètres composant le réseau de sorte que chacun ait été opéré à tour de rôle comme source de champs de référence, le procédé peut comporter une étape consistant à moyenner les positions et orientations d'un magnétomètre à localiser déterminées à l'issue des étapes de détermination, de manière à obtenir une meilleure estimation de la localisation du magnétomètre vectoriel.

Le lien entre la position d'un magnétomètre j dans un référentiel i et sa position dans un référentiel b peut être décrit comme suit : $\vec{p}_j^i = \mathbf{R}_i^b \vec{p}_j^b + \vec{p}_i^b$.

Ici $\vec{p}_j^i$ est le vecteur localisation du magnétomètre j dans le référentiel de la source i et $\mathbf{R}_i^b$ est la matrice de rotation qui décrit l'orientation du repère i décrit dans le repère b. Ainsi, on peut venir moyenner les vecteurs localisation $\vec{p}_j^i$ du magnétomètre j obtenus en ayant recours à différentes sources i, en venant exprimer ces différentes localisations dans un même référentiel b.

On peut trouver une estimation $\widehat{\vec{p}_i^b}$ du vecteur $\vec{p}_i^b$ et une estimation $\widehat{\mathbf{R}_i^b}$ de la matrice $\mathbf{R}_i^b$ qui minimisent l'erreur quadratique :

$$\left(\widehat{\mathbf{R}_i^b}, \widehat{\vec{p}_i^b}\right) = \underset{\mathbf{R}_i^b, \vec{p}_i^b}{argmin} \begin{pmatrix} \vec{p}_1^i - \mathbf{R}_i^b \vec{p}_1^b + \vec{p}_i^b \\ \vdots \\ \vec{p}_N^i - \mathbf{R}_i^b \vec{p}_k^b + \vec{p}_i^b \end{pmatrix}^T \begin{pmatrix} \vec{p}_1^i - \mathbf{R}_i^b \vec{p}_1^b + \vec{p}_i^b \\ \vdots \\ \vec{p}_N^i - \mathbf{R}_i^b \vec{p}_N^b + \vec{p}_i^b \end{pmatrix}$$

On peut également retrouver les angles d'Euler $\left(\widehat{\vec{\theta}_i^b}\right)$ ou bien le quaternion $\vec{q}_i^b$ qui représente la matrice $\mathbf{R}_i^b$ :

$$\left(\widehat{\vec{\theta}_i^b}, \widehat{\vec{p}_i^b}\right) = \underset{\vec{\theta}_i^b, \vec{p}_i^b}{argmin} \begin{pmatrix} \vec{p}_1^i - \mathbf{R}(\vec{\theta}_i^b) \vec{p}_1^b + \vec{p}_i^b \\ \vdots \\ \vec{p}_N^i - \mathbf{R}(\vec{\theta}_i^b) \vec{p}_i^b + \vec{p}_i^b \end{pmatrix}^T \begin{pmatrix} \vec{p}_1^i - \mathbf{R}(\vec{\theta}_i^b) \vec{p}_1^b + \vec{p}_i^b \\ \vdots \\ \vec{p}_k^i - \mathbf{R}(\vec{\theta}_i^b) \vec{p}_N^b + \vec{p}_i^b \end{pmatrix}$$

$$\left(\widehat{\vec{q}_i^b}, \widehat{\vec{p}_i^b}\right) = \underset{\vec{q}_i^b, \vec{p}_i^b}{argmin} \begin{pmatrix} \vec{p}_1^i - \mathbf{R}(\vec{q}_i^b) \vec{p}_1^b + \vec{p}_i^b \\ \vdots \\ \vec{p}_N^i - \mathbf{R}(\vec{q}_i^b) \vec{p}_N^b + \vec{p}_i^b \end{pmatrix}^T \begin{pmatrix} \vec{p}_1^i - \mathbf{R}(\vec{q}_i^b) \vec{p}_1^b + \vec{p}_i^b \\ \vdots \\ \vec{p}_k^i - \mathbf{R}(\vec{q}_i^b) \vec{p}_N^b + \vec{p}_i^b \end{pmatrix}$$

Il est également possible d'ajouter une pondération à cette minimisation afin de donner plus de poids aux magnétomètres qui donnent les estimations les plus précises. La précision de l'estimation dépend du rapport signal à bruit mesuré par un magnétomètre, les magnétomètres récepteurs les plus proches du magnétomètre émetteur étant mieux localisés que les magnétomètres récepteurs les plus éloignés. Pour cela, on utilise la matrice de pondération W mentionnée ci-après :

$$\left(\widehat{\vec{q}_i^b}, \widehat{\vec{p}_i^b}\right) = \underset{\vec{q}_i^b, \vec{p}_i^b}{argmin} \begin{pmatrix} \vec{p}_1^i - \mathbf{R}(\vec{q}_i^b) \vec{p}_1^b + \vec{p}_i^b \\ \vdots \\ \vec{p}_N^i - \mathbf{R}(\vec{q}_i^b) \vec{p}_N^b + \vec{p}_i^b \end{pmatrix}^T W \begin{pmatrix} \vec{p}_1^i - \mathbf{R}(\vec{q}_i^b) \vec{p}_1^b + \vec{p}_i^b \\ \vdots \\ \vec{p}_k^i - \mathbf{R}(\vec{q}_i^b) \vec{p}_N^b + \vec{p}_i^b \end{pmatrix}$$

Toujours dans le cadre du second mode de réalisation dans lequel l'un des magnétomètres du réseau est utilisé en tant que source de champ magnétique, les étapes de mesure et de détermination peuvent être mises en oeuvre par le ou les magnétomètres du réseau situés dans une zone d'émission prédéterminée autour de la source de champ magnétique. La zone d'émission couvre typiquement les magnétomètres les plus proches du magnétomètre source, par exemple ceux situés à un maximum de sauts donné du magnétomètre de référence dans le réseau (un magnétomètre à un saut correspondant à un magnétomètre immédiatement adjacent). Il est alors possible de procéder à la réitération des différentes étapes du procédé en utilisant comme nouveau magnétomètre source un magnétomètre présent dans ladite zone d'émission autour du magnétomètre utilisé comme source lors de l'itération précédente.

On peut de la sorte réaliser une localisation séquentielle qui permet de garantir une bonne précision de l'estimation, celle-ci dépendant effectivement du rapport signal à bruit et conduisant les magnétomètres les plus proches à être mieux localisés que les magnétomètres les plus éloignés. Cette localisation séquentielle permet une estimation de localisation de façon incrémentale, de proche en proche. En effet, on peut déterminer la position de tous les magnétomètres dans le référentiel de l'un des magnétomètres émetteurs de façon séquentielle selon $\vec{p}_j^i = \mathbf{R}_i^b \vec{p}_j^b + \vec{p}_i^b$, où $\mathbf{R}_i^b$ est la matrice de rotation du référentiel lié à la source i décrit dans le référentiel lié à la source b et $\vec{p}_i^b$ est le vecteur décrivant la position de la source i dans le référentiel lié à la source b. De façon séquentielle, on peut donc trouver les positions des capteurs de proche en proche, exprimées dans le référentiel lié à la source b.

Dans une variante avantageuse, le nouveau magnétomètre à utiliser comme source lors d'une nouvelle itération présente une zone d'émission qui se chevauche avec celle du magnétomètre utilisé comme source lors d'une itération précédente.

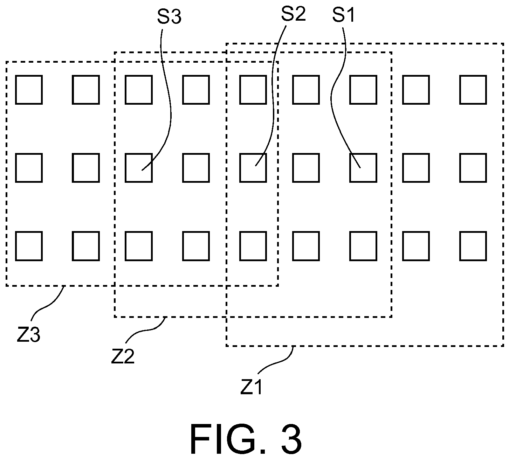

On a représenté sur la figure 3 un exemple de réseau avec zones successives d'émission chevauchantes. Sur cette figure, lors d'une première itération du procédé, un premier magnétomètre S1 est utilisé comme source avec une première zone d'émission Z1. On retrouve un deuxième magnétomètre S2 dans cette première zone d'émission Z1, et on vient déterminer sa localisation relativement au premier magnétomètre S1. Lors d'une deuxième itération du procédé, le deuxième magnétomètre S2 est utilisé comme source avec une deuxième zone d'émission Z2. On retrouve le premier magnétomètre S1 et un troisième magnétomètre S3 dans cette deuxième zone d'émission Z2, et on vient déterminer leurs localisations relativement au deuxième magnétomètre S2. Lors d'une troisième itération du procédé, le troisième magnétomètre S3 est utilisé comme source avec une troisième zone d'émission Z3. On retrouve le deuxième magnétomètre S2 dans cette troisième zone d'émission Z3, et on vient déterminer sa localisation relativement au troisième magnétomètre S3. En prévoyant des zones d'émission chevauchantes, on détermine pour le deuxième magnétomètre S2 à la fois sa localisation par rapport au premier magnétomètre S1 et sa localisation par rapport au troisième magnétomètre S3. Il s'avère de la sorte possible de mieux estimer la localisation du deuxième magnétomètre S2 par rapport aux différents magnétomètres source. Et comme présenté ci-dessus, il est possible de remonter de façon séquentielle à une localisation absolue, par exemple par rapport à un unique magnétomètre d'indice b du réseau, de chacun des magnétomètres du réseau. Par exemple, partant de la localisation du troisième magnétomètre S3 par rapport au deuxième magnétomètre S2, et connaissant la localisation du deuxième magnétomètre S2 par rapport au premier magnétomètre S1, on peut déterminer la localisation du troisième magnétomètre S3 par rapport au premier magnétomètre S1.

Ces localisations séquentielle ou par zones chevauchantes permettent de répondre au problème de la puissance d'émission limitée pouvant être générée par un magnétomètre source qui peut rendre impossible l'estimation de position du fait du bruit ambiant affectant la mesure par un magnétomètre de mesure. La mise en champ nul au moyen d'un blindage de l'appareil de mesure peut également être réalisée afin de supprimer ce bruit ambiant.

L'invention n'est pas limitée au procédé tel que précédemment décrit, mais s'étend également à un appareil de mesure du champ magnétique, tel qu'un appareil de magnétocardiographie ou de magnétoencéphalographie, et notamment un appareil comprenant :

- une source de champ magnétique configurée pour générer m champs magnétiques de référence, m étant un entier supérieur ou égal à 2, les amplitudes des m champs magnétiques étant connues et les directions des m champs magnétiques étant connues et distinctes ;
- au moins un magnétomètre vectoriel configuré pour mesurer les m champs magnétiques de référence suivant n axes, n étant un entier supérieur ou égal à 2 et m et n étant tels que m*n≥6 ; et
- un calculateur configuré pour déterminer la position et l'orientation, par rapport à la source de champ magnétique, de l'au moins un magnétomètre vectoriel à partir de la mesure des m champs magnétiques de référence sur les n axes de l'au moins un magnétomètre vectoriel.

On a vu précédemment que l'équation $\vec{B}_j^i = \vec{h}(\vec{x}_j^i)$ décrit la mesure du/des champs magnétique(s) de référence émis par la source i et mesuré(s) par le magnétomètre j et que l'on vient inverser la fonction $\vec{h}(\vec{x}_j^i)$ lors de l'étape de localisation pour estimer le vecteur d'état $\vec{x}_j^i$ à partir des mesures $\vec{B}_j^i$.

La solution à ce problème d'inversion n'est généralement pas déterminée de façon exacte à cause de la présence de bruit qui donne un caractère probabiliste aux mesures. On a donc recours à un estimateur afin de déterminer le vecteur d'état qui minimise un critère bien déterminé avec, en option, un modèle d'évolution qui permet de suivre la position des magnétomètres dans le temps. On introduit donc la notion de temps qui permettra par la suite d'ajouter un filtrage sur les mesures :

$$\vec{B}_j^i(t) = \vec{h}\left(\vec{x}_j^i(t)\right) + \vec{\omega}_j^i(t)$$

Ici, $\vec{\omega}_j^i$ est le bruit de mesure sur le champ émis par i et mesuré par j. C'est une variable aléatoire qui dépend des caractéristiques de l'émetteur et du récepteur. Ce bruit est souvent dominé par le bruit thermique.

Un premier exemple de technique possible pour réaliser cette inversion à un instant t est une minimisation de type moindre carré. Le but est ici de trouver la valeur $\widehat{\vec{x}}_j^i(t)$ qui minimise l'erreur quadratique entre la mesure réelle et la mesure estimée :

$$\widehat{\vec{x}}_j^i(t) = \underset{\vec{x}_j^i(t)}{argmin}\left(\left[\vec{h}\left(\vec{x}_j^i(t)\right) - \vec{B}_j^i(t)\right]^T \left[\vec{h}\left(\vec{x}_j^i(t)\right) - \vec{B}_j^i(t)\right]\right)$$

Comme cette fonction n'est pas linéaire, on peut utiliser des techniques d'optimisation tel que la descente de gradient, Gauss-Newton ou encore Levenberg-Marquardt.

Dans le cas particulier d'estimation de constellation de positions de magnétomètres fixes ou quasi-fixes, il est possible de considérer que la position $\widehat{\vec{x}_j^i}(t)$ ne varie pas dans une fenêtre de temps de n échantillons. Ceci permet de mieux filtrer les bruits des capteurs en moyennant l'information obtenue par les mesures aux instants $t_i$. La fonction à minimiser devient alors :

$$\widehat{\vec{x}_j^i} = \underset{\vec{x}_j^i}{argmin} \left( \left[ \begin{bmatrix} \vec{h}(\vec{x}_j^i(t_1)) \\ \vdots \\ \vec{h}(\vec{x}_j^i(t_n)) \end{bmatrix} - \begin{bmatrix} \vec{B}_j^i(t_1) \\ \vdots \\ \vec{B}_j^i(t_n) \end{bmatrix} \right]^T \left[ \begin{bmatrix} \vec{h}(\vec{x}_j^i(t_1)) \\ \vdots \\ \vec{h}(\vec{x}_j^i(t_n)) \end{bmatrix} - \begin{bmatrix} \vec{B}_j^i(t_1) \\ \vdots \\ \vec{B}_j^i(t_n) \end{bmatrix} \right] \right)$$

Selon un second exemple de technique d'inversion, on utilise un estimateur de maximum de vraisemblance qui cherche à trouver le $\widehat{\vec{x}_j^i}(t)$ qui maximise la probabilité d'obtenir les mesures réelles (i.e. les $\vec{B}_j^i$ ) :

$$\widehat{\vec{x}_j^i(t)} = \underset{\vec{x}_j^i(t)}{argmax} \, l\left(\widehat{\vec{x}_j^i}(t), \vec{B}_j^i(t)\right)$$

Ici l est la fonction de vraisemblance (« Likelyhood »).

Comme pour le cas précédent, il est possible de considérer que la position $\widehat{\vec{x}_j^i}(t)$ ne varie pas sur une fenêtre de temps de n échantillons. L'estimateur devient alors :

$$\widehat{\vec{x}_j^i} = \underset{\vec{x}_j^i}{argmax} \, l\left(\widehat{\vec{x}_j^i}, \vec{B}_j^i(t_1), \dots, \vec{B}_j^i(t_n)\right)$$

Dans cette technique, on utilise un cadre probabiliste. Il est donc possible d'introduire des informations physiques sur la qualité de la mesure donnée par les magnétomètres.

Dans un autre exemple de technique d'inversion, on utilise un modèle d'évolution qui permet de filtrer les mesures dans le temps et donner aux magnétomètres la possibilité de bouger dans le temps. Les algorithmes qui se prêtent à ce cadre sont souvent les algorithmes d'estimation bayésiennes, tels que les filtres de Kalman, ou encore les filtres à particules.

Par exemple, en utilisant un filtre de Kalman sous forme discrète et en prenant comme fonction d'évolution l'identité (on suppose que le capteur peut bouger d'une façon aléatoire (gaussienne) autour de sa position à l'instant précédent, on part du système d'état suivant :

$$\vec{x}_j^i(t_k) = \vec{x}_j^i(t_{k-1}) + \vartheta_j^i(t_{k-1})$$

$$\vec{B}_j^i(t_k) = \vec{h}\left(\vec{x}_j^i(t_k)\right) + \omega_j^i(t_k)$$

Ici $\vartheta_j^i(t_{k-1})$ est le bruit d'état qui décrit l'incertitude sur le mouvement du magnétomètre entre l'instant $t_{k-1}$ et l'instant $t_k$ (on considère qu'il s'agit d'un bruit Gaussien centré de matrice de covariance $\mathbf{Q}_j^i(t_{k-1})$). $\omega_j^i(t_k)$ est le bruit de mesure qui décrit le bruit qui entache la mesure (on considère qu'il s'agit d'un bruit Gaussien centré de matrice de covariance $\mathbf{R}_j^i(t_k)$).

Le filtre de Kalman permet d'estimer de façon optimale (dans le cas où la fonction est linéaire et le bruit est gaussien) le vecteur d'état $\vec{x}_j^i(t_k)$ à chaque instant $t_k$ tout en filtrant cette estimation en utilisant des informations accumulées du passé et des probabilités décrivant l'évolution de l'état dans le temps. Le filtre de Kalman traite le vecteur d'état comme une variable aléatoire gaussienne décrite par une espérance $\widehat{\vec{x}_j^i}(t_k)$ et une matrice de covariance $\widehat{\mathbf{P}_j^i}(t_k)$.

Une estimation initiale de l'état est requise. Il s'agit d'une variable aléatoire puisqu'on ne connait pas les positions des magnétomètres de façon très précise au début de l'expérience. Cette initialisation est une connaissance à priori des positions des magnétomètres. Elle est décrite par une espérance $\widehat{\vec{x}_j^i}(t_0)$ et une matrice de covariance $\widehat{\mathbf{P}_j^i}(t_0)$ qui donne une information sur l'incertitude qu'on a sur cette position initiale.

A chaque instant de mesure $t_k$, les magnétomètres mesurent les champs magnétiques émis par la source et estiment leur état en deux étapes. La première étape appelée prédiction, utilise la première équation d'état afin d'estimer l'état actuel en fonction de l'état précèdent :

$$\vec{x}_j^i(t_k)^* = \vec{x}_j^i(t_{k-1})$$

$$\widehat{\mathbf{P}_j^i}(t_k)^* = \widehat{\mathbf{P}_j^i}(t_{k-1}) + \mathbf{Q}_j^i(t_{k-1})$$

La deuxième phase de l'algorithme consiste à mettre à jour cette estimation en utilisant la mesure réalisée à l'instant $t_k$. Cette correction s'effectue comme suit :

$$\vec{x}_j^i(t_k) = \vec{x}_j^i(t_k)^* + \mathbf{K}\vec{y}_j^i(t_k)$$

$$\widehat{\mathbf{P}_j^i}(t_k) = \left(\mathbf{I} - \mathbf{K}_j^i(t_k)\mathbf{H}\left(\vec{x}_j^i(t_k)\right)\right)\widehat{\mathbf{P}_j^i}(t_k)^*$$

Avec H la matrice jacobéenne de la fonction $\vec{h}$. $\vec{y}_j^i(t_k)$ est l'innovation apportée par la mesure à l'instant $t_k$ :

$$\vec{y}_j^i(t_k) = \vec{B}_j^i(t_k) - \vec{h}\left(\vec{x}_j^i(t_k)\right)$$

$\mathbf{K}_j^i(t_k)$ est le gain de Kalman calculé comme suit :

$$\mathbf{K}_j^i(t_k) = \widehat{\mathbf{P}_j^i}(t_k)^*\mathbf{H}^T\left(\vec{x}_j^i(t_k)\right)\mathbf{S}_j^{i^{-1}}(t_k)$$

$\mathbf{S}_j^i(t_k)$ est la matrice de covariance de l'innovation calculée comme suit :

$$\mathbf{S}_j^i(t_k) = \mathbf{H}\left(\vec{x}_j^i(t_k)\right)\widehat{\mathbf{P}_j^i}(t_k)^*\mathbf{H}^T\left(\vec{x}_j^i(t_k)\right) + \mathbf{R}_j^i(t_k)$$

**Revendications**

1. Procédé de localisation d'au moins un magnétomètre vectoriel (C1-C3) appartenant à un réseau de magnétomètres vectoriels, comprenant les étapes suivantes :

- contrôle de l'un des magnétomètres vectoriels du réseau dit magnétomètre source pour qu'il génère (EMi) m champs magnétiques de référence, m étant un entier supérieur ou égal à 2, les amplitudes des m champs magnétiques étant connues et les directions des m champs magnétiques étant connues et distinctes ;

- mesure (MESj), par au moins un des autres magnétomètres du réseau dit magnétomètre de mesure, des m champs magnétiques de référence suivant n axes de l'au moins un magnétomètre de mesure (C1-C3), n étant un entier supérieur ou égal à 2 et m et n étant tels que m*n≥6 ;

- détermination (LOCj) de la position et de l'orientation, par rapport au magnétomètre source, de l'au moins un magnétomètre de mesure (C1-C3) à partir de la mesure des m champs magnétiques de référence sur les n axes de l'au moins un magnétomètre de mesure.

2. Procédé selon la revendication 1, dans lequel les m champs magnétiques de référence sont émis simultanément, les amplitudes des m champs magnétiques étant distinctes.

3. Procédé selon la revendication 1, dans lequel les m champs magnétiques de référence sont émis séquentiellement.

4. Procédé selon la revendication 3, dans lequel l'étape de contrôle du magnétomètre source comprend un créneau temporel de non-génération d'un champ magnétique de référence, une étape de mesure d'un champ magnétique ambiant suivant les n axes de l'au moins un magnétomètre de mesure est réalisée au cours dudit créneau temporel, et l'étape de détermination comprend une soustraction de la mesure du champ magnétique ambiant à la mesure des m champs magnétiques de référence.

5. Procédé selon la revendication 1, dans lequel le magnétomètre source réalise une modulation fréquentielle des m champs magnétiques de référence.

6. Procédé selon la revendication 5, dans lequel le magnétomètre source réalise un multiplexage fréquentiel des m champs magnétiques de référence.

7. Procédé selon l'une des revendications 1 à 6, dans lequel le magnétomètre source comprend m bobines et un générateur de fréquence permettant d'injecter un courant d'amplitude connue dans chacune des m bobines.

8. Procédé selon l'une des revendications 1 à 7, comprenant une réitération des étapes de contrôle, mesure et détermination en utilisant pour magnétomètre source un autre magnétomètre vectoriel dudit réseau.

9. Procédé selon la revendication 8, comprenant en outre une étape consistant à moyenner les positions et orientations de l'au moins un magnétomètre de mesure déterminées à l'issue des étapes de détermination.

10. Procédé selon la revendication 8, dans lequel les étapes de mesure et de détermination sont mises en oeuvre par le ou les magnétomètres du réseau situés dans une zone d'émission autour du magnétomètre source, et dans lequel ledit autre magnétomètre vectoriel est choisi parmi ledit ou lesdits magnétomètres du réseau situés dans la zone d'émission autour du magnétomètre source.

11. Procédé selon la revendication 10, dans lequel la zone d'émission autour du magnétomètre source et la zone d'émission dudit autre magnétomètre vectoriel se chevauchent.

12. Procédé selon l'une des revendications 1 à 11 dans lequel les magnétomètres vectoriels sont portés par une structure conformable et comprenant une étape préalable d'installation de la structure conformable sur un utilisateur.

13. Appareil de mesure du champ magnétique, comprenant :

- un réseau de magnétomètres vectoriels comportant :

o un magnétomètre source (S) apte à être contrôlé pour générer m champs magnétiques de référence, m étant un entier supérieur ou égal à 2, les amplitudes des m champs magnétiques étant connues et les directions des m champs magnétiques étant connues et distinctes ;

o au moins un magnétomètre de mesure (C1-C3) configuré pour mesurer les m champs magnétiques de référence suivant n axes de l'au moins un magnétomètre de mesure, n étant un entier supérieur ou égal à 2 et m et n étant tels que m*n≥6 ; et

- un calculateur configuré pour déterminer la position et l'orientation, par rapport au magnétomètre source, de l'au moins un magnétomètre de mesure à partir de la mesure des m champs magnétiques de référence sur les n axes de l'au moins un magnétomètre de mesure.

**Patentansprüche**

1. Verfahren zur Lokalisierung wenigstens eines vektoriellen Magnetometers (C1-C3), das zu einem Netz von vektoriellen Magnetometern gehört, umfassend die folgenden Schritte:

   - Steuern von einem der vektoriellen Magnetometer des Netzes, genannt Quellenmagnetometer, damit es m Referenzmagnetfelder generiert (EMi), wobei m eine ganze Zahl größer oder gleich 2 ist, wobei die Amplituden der m Magnetfelder bekannt sind, und wobei die Richtungen der m Magnetfelder bekannt und verschieden sind;
   - Messen (MESj), durch wenigstens eines der anderen Magnetometer des Netzes, genannt Messmagnetometer, der m Referenzmagnetfelder entlang n Achsen des wenigstens einen Messmagnetometers (C1-C3), wobei n eine ganze Zahl größer oder gleich 2 ist und wobei m und n derart sind, dass m*n $\geq$ 6;
   - Bestimmen (LOCj) der Position und der Orientierung, bezogen auf das Quellenmagnetometer, des wenigstens einen Messmagnetometers (C1-C3) ausgehend von der Messung der m Referenzmagnetfelder auf den n Achsen des wenigstens einen Messmagnetometers.

2. Verfahren nach Anspruch 1, bei dem die m Referenzmagnetfelder gleichzeitig emittiert werden, wobei die Amplituden der m Magnetfelder verschieden sind.

3. Verfahren nach Anspruch 1, wobei die m Referenzmagnetfelder sequentiell emittiert werden.

4. Verfahren nach Anspruch 3, wobei der Schritt des Steuerns des Quellenmagnetometers ein Zeitfenster umfasst, in dem kein Referenzmagnetfeld generiert wird, wobei ein Schritt des Messens eines Umgebungsmagnetfelds entlang den n Achsen des wenigstens einen Messmagnetometers im Verlauf des Zeitfensters durchgeführt wird, und wobei der Schritt des Bestimmens ein Subtrahieren der Messung des Umgebungsmagnetfelds von der Messung der m Referenzmagnetfelder umfasst.

5. Verfahren nach Anspruch 1, wobei das Quellenmagnetometer eine Frequenzmodulation der m Referenzmagnetfelder durchführt.

6. Verfahren nach Anspruch 5, wobei das Quellenmagnetometer eine Frequenzmultiplexierung der m Referenzmagnetfelder durchführt.

7. Verfahren nach einem der Ansprüche 1 bis 6, wobei das Quellenmagnetometer m Spulen und einen Frequenzgenerator umfasst, der es ermöglicht, einen Strom mit bekannter Amplitude in jede der m Spulen zu injizieren.

8. Verfahren nach einem der Ansprüche 1 bis 7, umfassend eine Reiteration der Schritte der Steuerung, der Messung und der Bestimmung unter Verwendung eines anderen vektoriellen Magnetometers des Netzes als Quellenmagnetometer.

9. Verfahren nach Anspruch 8, ferner umfassend einen Schritt, der darin besteht, die Positionen und Orientierungen des wenigstens einen Messmagnetometers zu mitteln, die am Ende der Schritte der Bestimmung bestimmt worden sind.

10. Verfahren nach Anspruch 8, wobei die Schritte des Messens und der Bestimmung durch das oder die Magnetometer des Netzes durchgeführt werden, die in einer Emissionszone um das Quellenmagnetometer herum angeordnet sind, und wobei das andere vektorielle Magnetometer aus dem oder den Magnetometern des Netzes ausgewählt wird, die in der Emissionszone um das Quellenmagnetometer herum angeordnet sind.

11. Verfahren nach Anspruch 10, wobei die Emissionszone um das Quellenmagnetometer herum, und die Emissionszone des anderen vektoriellen Magnetometers sich überlappen.

12. Verfahren nach einem der Ansprüche 1 bis 11, bei dem die vektoriellen Magnetometer durch eine anpassbare Struktur getragen werden, und umfassend einen vorherigen Schritt des Installierens der anpassbaren Struktur an einen Benutzer.

**13.** Gerät zur Magnetfeldmessung, umfassend:

- ein Netz von vektoriellen Magnetometern, umfassend:

o ein Quellenmagnetometer (S), das dazu ausgelegt ist, zur Generierung von m Referenzmagnetfeldern gesteuert zu werden, wobei m eine ganze Zahl größer oder gleich 2 ist, wobei die Amplituden der m Magnetfelder bekannt sind, und wobei die Richtungen der m Magnetfelder bekannt und verschieden sind; o wenigstens ein Messmagnetometer (C1-C3), das dazu konfiguriert ist, die m Referenzmagnetfelder entlang n Achsen des wenigstens einen Messmagnetometers zu messen, wobei n eine ganze Zahl größer oder gleich 2 ist, und wobei m und n derart sind, dass $m*n \geq 6$; und

- einen Rechner, der dazu konfiguriert ist, die Position und die Orientierung des wenigstens einen Messmagnetometers, bezogen auf das Quellenmagnetometer, ausgehend von der Messung der m Referenzmagnetfelder auf den n Achsen des wenigstens einen Messmagnetometers zu bestimmen.

**Claims**

**1.** Method for localisation of at least one vector magnetometer (C1-C3) of a network of vector magnetometers, comprising the following steps:

- control of one of the vector magnetometers of the network, called source magnetometer, so that it generates (EMi) m reference magnetic fields, where m is an integer greater than or equal to 2, the amplitudes of the m magnetic fields being known and the directions of the m magnetic fields being known and distinct;
- measurement (MESj), by at least one of the other magnetometers of the network, called measurement magnetometer, of the m reference magnetic fields along n axes of the at least one measurement magnetometer (C1-C3), where n is an integer greater than or equal to 2 and m and n are such that $m*n \geq 6$;
- determination (LOCj) of the position and orientation of the at least one measurement magnetometer (C1-C3) relative to the source magnetometer, starting from the measurement of the m reference magnetic fields on the n axes of the at least one measure magnetometer.

**2.** Method according to claim 1, in which the m reference magnetic fields are emitted simultaneously, the amplitudes of the m magnetic fields being distinct.

**3.** Method according to claim 1, in which the m reference magnetic fields are emitted sequentially.

**4.** Method according to claim 3, in which the control step of the source magnetometer includes a time slot in which a reference magnetic field is not generated, a step to measure an ambient magnetic field along the n axes of the at least one measurement magnetometer is done during said time slot, and the determination step includes subtraction of the measurement of the ambient magnetic field from the measurement of the m reference magnetic fields.

**5.** Method according to claim 1, in which the source magnetometer makes a frequency modulation of the m reference magnetic fields.

**6.** Method according to claim 5, in which the source magnetometer makes a frequency multiplexing of the m reference magnetic fields.

**7.** Method according to one of claims 1 to 6, in which the source magnetometer comprises m coils and a frequency generator to inject a current with known amplitude into each of the m coils.

**8.** Method according to one of claims 1 to 7, comprising a reiteration of the control, measurement and determination steps, using another vector magnetometer in said network as the source magnetometer.

**9.** Method according to claim 8, also comprising a step consisting of averaging the positions and orientations of the at least one measurement magnetometer determined after the determination steps.

**10.** Method according to claim 8, in which the measurement and determination steps are implemented by the magnetometer(s) in the network located in an emission zone around the source magnetometer, and in which said other vector magnetometer is chosen from among said magnetometer(s) in the network located in the emission zone around the source magnetometer.

**11.** Method according to claim 10, in which the emission zone around the source magnetometer and the emission zone of said other magnetometer overlap.

**12.** Method according to one of claims 1 to 11, in which the vector magnetometers are carried by a conformable structure and including a prior step to install the conformable structure on a user.

**13.** Magnetic field measurement instrument, comprising:

- a network of magnetometers having:

  ∘ a source magnetometer (S) configured to generate m reference magnetic fields, where m is an integer greater than or equal to 2, the amplitudes of the m magnetic fields being known and the directions of the m magnetic fields being known and distinct;
  ∘ at least one measurement magnetometer (C1-C3) configured to measure the m reference magnetic fields along n axes of the at least one measurement magnetometer, where n is an integer greater than or equal to 2 and m and n are such that $m*n \geq 6$; and

- a computer configured to determine the position and orientation of the at least one measurement magnetometer relative to the source magnetometer, starting from the measurement of the m reference magnetic fields on the n axes of the at least one measurement magnetometer.

FIG. 1

FIG. 2

FIG. 3